# EUROPEAN PATENT SPECIFICATION

(11) **EP 2 257 272 B1**
(45) Date of publication and mention of the grant of the patent: **25.09.2013**
(21) Application number: 09728453.3
(22) Date of filing: 25.03.2009
(51) Int. Cl.: A61K 8/02, A61K 8/98, A61Q 19/08

(54) **METHOD FOR THE COSMETIC TREATMENT OF SKIN PHOTO-AGEING**
VERFAHREN ZUR KOSMETISCHEN BEHANDLUNG VON HAUTBEHANDLUNG BEI LICHTALTERUNG
PROCÉDÉ DE TRAITEMENT COSMÉTIQUE DU PHOTO-VIEILLISSEMENT DE LA PEAU

(30) Priority: 31.03.2008 US 40891 P
(43) Date of publication of application: 08.12.2010
(73) Proprietor: Scarcell Therapeutics, 75008 Paris (FR)
(72) Inventor: GOGLY, Bruno, F-77510 Hondevilliers (FR); LAFONT, Antoine, F-75007 Paris (FR); COULOMB, Bernard, F-91430 Igny (FR)
(74) Representative: Domenego, Bertrand
(86) International application number: PCT/EP2009/053474
(87) International publication number: WO 2009/121761

(56) References cited:
- WO-A-2008/017927
- FR-A- 2 872 431
- US-A1- 2008 050 346
- EBISAWA K. ET AL.: "The Expression Profile of Anti-Aging Related Genes in Dermal and Gingival Fibroblasts" ANN. NUTR. METAB., vol. 51, 1 November 2007 (2007-11-01), page 409, XP002535647

## Description

In skin ageing, disequilibrium occurs in the balance between synthesis of the extracellular matrix (ECM) and its degradation by matrix metalloproteases (MMPs). This disequilibrium leads to an excessive degradation of the extracellular matrix, a characteristic of skin ageing (Cauchard & Homebeck (2004) Vivant 5)*.* Skin ageing is associated to an increase in the number and the deepness of wrinkles, a direct consequence of the degradation of macromolecules of the dermis, such as collagens and elastin.

In dermis, MMP overproduction which occurs in chronological and photo-induced ageing is stimulated by oxygenated free radicals. Besides, in skin areas exposed to sun, such as facial skin, other deleterious effects of UV rays occur, in particular incomplete collagen synthesis, skin pigmentation, and solar elastosis (which presents as a degradation of the cutaneous elastic lattice). Furthermore, in *vitro* studies have shown that MMP are overproduced by skin fibroblasts submitted to UV-ray treatment (Brennan et al. (2003) Photochem. Photobiol. 78:43-48)

Collagenases 1 and 3 (MMP-1 et MMP-13) and MT1-MMP (MMP-14) degrade collagens, while gelatinases A and B (MMP-2 and MMP-9) degrade elastin. Other metalloproteinases such as stromelysin 1 (MMP-3) are involved both in collagen and elastin degradation.

Dermal fibroblasts have been used in the frame of the treatment of skin ageing (Weiss et al. (2007) Dermatol Surg. 33:263-8). An increase of the collagen lattice could be observed in 215 subjects injected autologous dermal fibroblasts (20 millions/ml) in deep wrinkles. The improvement in wrinkles was still clearly visible on 80% of the subjects, one year after injection.

Gingival fibroblasts are mesenchymal cells which are capable of migrating, adhering and proliferating within the soft connective tissues of the gum, thereby maintaining the integrity of the gingival tissue which is exposed to numerous aggressions, such as mechanical stresses, bacterial infections, or pH and temperature variations. Gingival fibroblasts are in particular described in Gogly et al., (1997) Clin. Oral Invest. 1:147-152; Gogly et al. (1998) Biochem. Pharmacol. 56:1447-1454; and Ejeil et al. (2003) J. Periodontol. 74:188-195.

Depending on environmental conditions, gingival fibroblasts are capable to modulate their phenotype, and to respond by proliferating, migrating, synthesising matrix components or matrix-related enzymes.

Gingival fibroblasts synthesise collagens (*e.g.* types I, III, V, VI, VII, XII), elastic fibers (oxytalan, elaunin and elastin), proteoglycans and glycosaminoglycans (e.g. decorin, biglycan), and glycoproteins (e.g. fibronectin, tenascin). Simultaneously, gingival fibroblasts synthesise enzymes that are able to degrade the macromolecular compounds (matrix metalloproteinases; MMPs), but also enzymes inhibiting active forms of MMPs (Inhibitors of metalloproteinases; TIMPs). Gingival fibroblasts are thus important actors of extracellular matrix remodelling.

### Summary of the invention

The present invention arises from the unexpected finding, by the inventors, that gingival fibroblasts are more suited than dermal fibroblasts for inhibiting MMP activity originating from UV-treated dermal fibroblasts.

Thus, the present invention relates to a non-therapeutic method for the prevention or treatment of skin photo ageing in an individual, comprising administering to said individual a cosmetically active quantity of a gingival fibroblast-derived product.

### Description of the figures

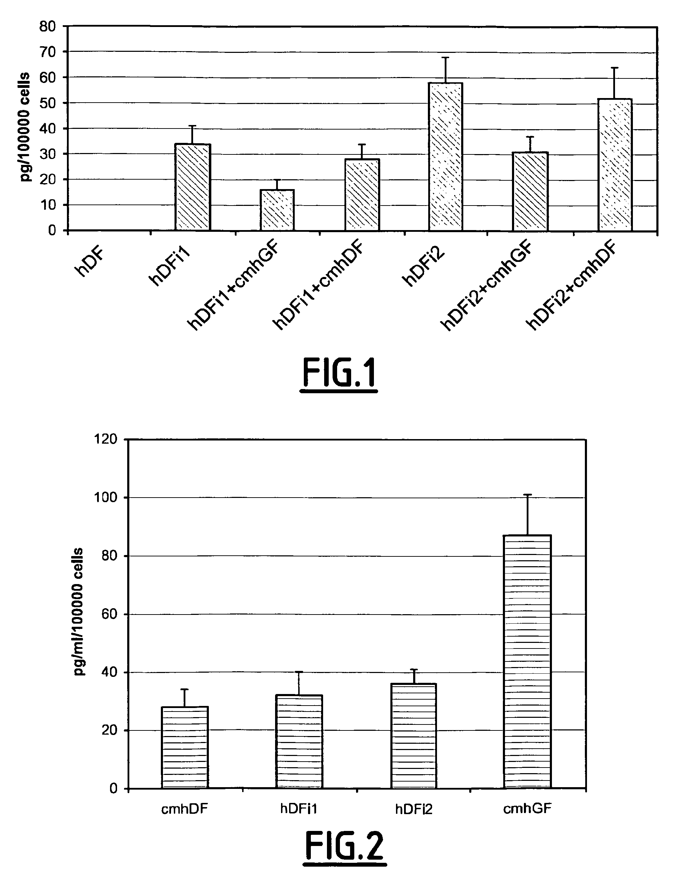

Figure 1 represents the quantity of MMP-9 (vertical axis, pg/100,000 cells) in the culture medium of: untreated human dermal fibroblasts (hDF); 7.5 Joules/cm² UV-A-treated human dermal fibroblasts (hDFi1); hDFi1 in the presence of human gingival fibroblast conditioned medium (cmhGF); hDFi1 in the presence of human dermal fibroblast conditioned medium (cmhDF); 15 Joules/cm² UV-A-treated human dermal fibroblasts (hDFi2); hDFi2 in the presence of human gingival fibroblast conditioned medium (cmhGF); and hDFi2 in the presence of human dermal fibroblast conditioned medium (cmhDF).

Figure 2 represents the concentration of TIMP-1 (vertical axis, pg/ml/100,000 cells) in human dermal fibroblast conditioned medium (cmhDF), in the culture medium of UV-A-treated human dermal fibroblast at 7.5 Joules/cm² (hDFi1) or 15 Joules/cm² (hDFi2), or in human gingival fibroblast conditioned medium (cmhGF).

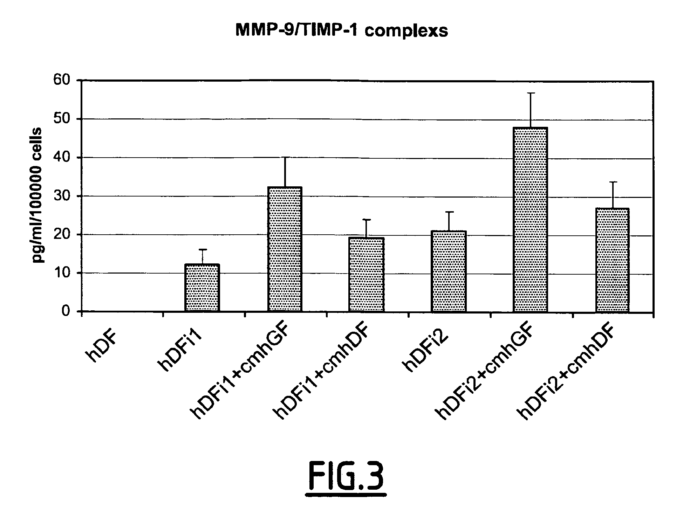

Figure 3 represents the concentration of MMP-9/TIMP-1 complexes (vertical axis, pg/ml/100,000 cells) in the culture medium of: untreated human dermal fibroblasts (hDF); 7.5 Joules/cm² UV-A-treated human dermal fibroblasts (hDFi1); hDFi1 in the presence of human gingival fibroblast conditioned medium (cmhGF); hDFi1 in the presence of human dermal fibroblast conditioned medium (cmhDF); 15 Joules/cm² UV-A-treated human dermal fibroblasts (hDFi2); hDFi2 in the presence of human gingival fibroblast conditioned medium (cmhGF); and hDFi2 in the presence of human dermal fibroblast conditioned medium (cmhDF).

### Detailed description of the invention

As intended herein "skin ageing" relates to skin defects which occur as a consequence of a degradation of skin constituents due to chronic factors, such as mechanical, oxidative and/or photo stresses.

In particular, skin aging can be a consequence of chronological ageing and/or photo-ageing. "Chronological ageing" relates to skin defects which occur as a consequence oldness. "Photo-ageing" relates to skin defects which occur as a consequence of skin exposition to light, and in particular to UV rays, more particularly UV-A rays.

The skin defects can notably be wrinkles or loss of skin elasticity. The degraded skin constituents can be elastin and/or collagens, which the method according to the invention is useful for increasing synthesis thereof within dermis.

The method of the invention is for the prevention or treatment of skin photo ageing.

Preferably the individual is a mammal and more preferably a human.

Procedures for taking, culturing and preserving gingival fibroblasts are well known to the man skilled in the art and are particularly described in Naveau et al. (2006) J. Periodontol. 77:238-47 and in Gogly et al. (2007) Arterioscler. Thromb. Vasc. Biol. 27:1984-90.

Advantageously, gingival fibroblasts are easily sampled and cultured. Besides, gingival fibroblasts possess a high expansion rate.

Preferably, the gingival fibroblasts used in the method according to the invention are autologous, that is they are taken from the individual, to whom the gingival fibroblast-derived product is intended to be administered.

Advantageously, gingival fibroblasts provide for an almost limitless source of autologous fibroblasts. Furthermore, in case of aged skin, culture-competent autologous gingival fibroblasts are usually still available, whereas, in contrast, sources of culture-competent autologous dermal fibroblasts are scarce.

However, the gingival fibroblasts can also be allogenic, that is taken from another individual of the same species or heterologous, that is taken from another individual of another species.

As intended herein "gingival fibroblast-derived product" relates to any product which can be obtained from gingival fibroblasts in themselves or which contains gingival fibroblasts secretions. For example, it is preferred that the gingival fibroblast derived product is selected from the group consisting of gingival fibroblast whole cells, a gingival fibroblast culture, a gingival fibroblast extract, and a gingival fibroblast conditioned medium.

Gingival fibroblast extracts can be obtained by any cell fragmentation method known in the art.

Gingival fibroblast conditioned medium relates to any medium, such as a liquid cell culture medium, which has been contacted by gingival fibroblasts, in particular for a time sufficient for the gingival fibroblasts to have secreted in the medium.

Administration of the gingival fibroblast-derived product, preferably at a site near the skin area to be treated, can proceed by any method known in the art. However, it is preferred that the gingival fibroblast-derived product is administered topically or by intradermal injection. Such administration routes are well known to anyone of skill in the art and are notably described by Weiss et al. (2007) Dermatol Surg. 33:263-8.

Preferably, the method according to the invention comprises the following steps:
- taking gingival fibroblasts from the individual;
- culturing the gingival fibroblasts;
- obtaining a gingival fibroblast-derived product from the cultured gingival fibroblasts;
- administering the gingival fibroblast-derived product to the individual.

### EXAMPLE

### Methods

### 1. Cell Culture

Five human gingival fibroblast (hGF) and three dermal fibroblast (hDF) cultures were obtained from gingival and dermal explants of healthy patients (20-30 years old). Primary explant cultures were established and used from passage 3 to 5.

### Preparation of hGF or hDF conditioned medium

The culture medium (DMEM/FCS) from 75 cm² flasks of confluent hGF and hDF cultures, was discarded. 24 ml of DMEM was then added and retrieved 24 hours later. Conditioned medium was then freezed until use.

### Preparation of cells

Three 12-wells plates were seeded with hDF from two 25 cm2 flasks at confluence. When confluence was reached (150,000 cells per well), 2 plates were UVA-irradiated respectively at 7.5 and 15 joules/cm², the third plate was used as a control, to check for the absence of MMP-9 in absence of irradiation.

The culture media were changed after irradiation. For each flask, the following media were added:
- DMEM only for 4 wells (1ml per well)
- hGF conditioned medium for 4 wells (1 ml per well)
- hDF conditioned medium for 4 wells (1 ml per well)

Culture media were then collected 24 h later, aliquoted and stored at -80°C for further protein secretion analysis. Cells were fixed in the wells and GIEMSA stained.

### 2. MMP-9 and TIMP-1 secretion analysis

### Gelatin zymography (MMP-9)

Gelatin zymographies were performed on 20 µl of culture medium. 10 µl of pro-MMP-9 (92 kDa) and 10µl of pro-MMP-2 (72 kDa) (10 ng) (BC058 and BC057; ABCys) were ran on the same gel in order to facilitate the identification of the MMP types. Furthermore, 10 µl of pro-MMP-9 incubated with APMA (2 mM) at 37°C for 1 hour was ran in parallel to visualize MMP-9 position.

### Dot blotting (MMP-9 and TIMP-1)

10µl of culture media were applied onto nitrocellulose membrane. Membranes were then treated with primary anti-MMP-9 (free form) and anti-TIMP-1 (IM37 and IM32, respectively; Calbiochem) monoclonal mouse antibodies at a 1/500 dilution. Following washing in TBS/Tween (50mM Tris, 150mM NaCl, 0.1% Tween 20, pH 7.5), membranes were incubated with a peroxydase-labelled goat anti-mouse secondary antibody (1/1000, DC08L; Calbiochem) for 1 hour. Immunoreactive proteins visualized on Kodak Biomax MR films. The size of the blot (surface area) and grey intensities were analysed using Image J software (Image J; http:/rsb.info.nih.gov/ij/index.html). Concentration was determined by comparison with 10pg MMP-9 or TIMP-1 standards (PF140 and PF019, respectively; Calbiochem).

Complementary quantitative analysis of free MMP-9 and TIMP-1 were made by ELISA (DMP900 and DTM100; R&D Systems).

Statistical analysis between the different experiments was performed using Paired Student's t-test.

### 3. MMP-9/TIMP-1 complexes determination

Total human MMP-9/TIMP-1 complexes were quantified, using an enzyme-linked immunosorbent assay kit (ELISA) (DY1449; R&D Systems).

### Results

### 1. Conditioned medium from human gingival fibroblasts inhibits MMP-9 from UV-irradiated human dermal fibroblasts

**Figure 1** shows that human dermal fibroblasts (hDF) do not produce MMP-9 except after irradiation by UV-A at 7.5 joules/cm2 (hDFi1) or 15 joules/cm2 (hDFi2). A human gingival fibroblast conditioned medium (cmhGF) reduces MMP-9 production by UV treated-dermal fibroblasts by 50%, while a human dermal fibroblast conditioned medium (cmhDF) reduces MMP-9 production by only 15 %.

### 2. Human gingival fibroblasts produce more TIMP-1 (MMP-9 tissular inhibitor) than human dermal fibroblasts

**Figure 2** shows that hGF conditioned medium of contains at least 3 times more TIMP-1 than that of hDF, irradiated or not.

### 3. Increase in the quantity of MMP-9/TIMP-1 complexes in the presence of human gingival fibroblasts

**Figure 3** shows that the quantity TIMP-1/MMP-9 complexes is twice as important in the presence of cmhGF as in the presence of cmhDF.

## Claims

1. A non-therapeutic method for the prevention or treatment of skin photo-ageing in an individual, comprising administering to said individual a cosmetically active quantity of a gingival fibroblast-derived product selected from the group consisting of gingival fibroblast whole cells, a gingival fibroblast culture and a gingival fibroblast conditioned medium.

2. The method according to claim 1, for the cosmetic prevention or treatment of wrinkles or loss of skin elasticity.

3. The method according to claim 1 or 2, for the prevention or treatment of facial skin photo-ageing.

4. The method according to any of claims 1 to 3, for increasing elastin and/or collagen synthesis within dermis.

5. The method according to any of claims 1 to 4, wherein the gingival fibroblast-derived product is administered topically or by intradermal injection.

6. The method according to any of claims 1 to 5, wherein the gingival fibroblast-derived product is a gingival fibroblast conditioned medium.

7. The method according to any of claims 1 to 6, wherein the gingival fibroblast-derived product is obtained from gingival fibroblasts taken from the individual.

8. The method according to any of claims 1 to 7, comprising:
- taking gingival fibroblasts from the individual;
- culturing the gingival fibroblasts;
- obtaining a gingival fibroblast-derived product from the cultured gingival fibroblasts, said gingival fibroblast-derived product being selected from the group consisting of gingival fibroblast whole cells, a gingival fibroblast culture and a gingival fibroblast conditioned medium; and
- administering the gingival fibroblast-derived product to the individual.

## Patentansprüche

1. Nicht-therapeutisches Verfahren zur Prävention oder Behandlung von Lichtalterung der Haut bei einem Individuum, umfassend das Verabreichen einer kosmetisch wirksamen Menge eines von Gingivafibroblasten abgeleiteten Produkts, ausgewählt aus der Gruppe, bestehend aus ganzen Gingivafibroblastenzellen, einer Gingivafibroblastenkultur und einem konditionierten Gingivafibroblastenmedium, an das Individuum.

2. Verfahren nach Anspruch 1 zur kosmetischen Prävention oder Behandlung von Falten oder Verlust der Hautelastizität.

3. Verfahren nach Anspruch 1 oder 2 zur Prävention oder Behandlung von Lichtalterung der Gesichtshaut.

4. Verfahren nach einem der Ansprüche 1 bis 3 zum Steigern der Elastin- und/oder Kollagensynthese innerhalb der Dermis.

5. Verfahren nach einem der Ansprüche 1 bis 4, wobei das von Gingivafibroblasten abgeleitete Produkt topisch oder durch intradermale Injektion verabreicht wird.

6. Verfahren nach einem der Ansprüche 1 bis 5, wobei das von Gingivafibroblasten abgeleitete Produkt ein konditioniertes Gingivafibroblastenmedium ist.

7. Verfahren nach einem der Ansprüche 1 bis 6, wobei das von Gingivafibroblasten abgeleitete Produkt aus Gingivafibroblasten erhalten wird, die dem Individuum entnommen wurden.

8. Verfahren nach einem der Ansprüche 1 bis 7, umfassend:
- Entnehmen von Gingivafibroblasten aus dem Individuum;
- Züchten der Gingivafibroblasten;
- Erhalten eines von Gingivafibroblasten abgeleiteten Produkts aus den gezüchteten Gingivafibroblasten, wobei das genannte von Gingivafibroblasten abgeleitete Produkt aus der Gruppe, bestehend aus ganzen Gingivafibroblastenzellen, einer Gingivafibroblastenkultur und einem konditionierten Gingivafibroblastenmedium, ausgewählt ist; und
- Verabreichen des von Gingivafibroblasten abgeleiteten Produkts an das Individuum.

## Revendications

1. Procédé non thérapeutique de prévention ou de traitement du photovieillissement cutané chez un individu, comprenant l'administration audit individu d'une quantité cosmétiquement active d'un produit dérivé de fibroblastes gingivaux choisi parmi le groupe comprenant des cellules entières de fibroblastes gingivaux, une culture de fibroblastes gingivaux et un milieu conditionné de fibroblastes gingivaux.

2. Procédé selon la revendication 1, destiné à la prévention ou au traitement cosmétique des rides ou de la perte d'élasticité de la peau.

3. Procédé selon la revendication 1 ou 2, destiné à la prévention ou au traitement du photovieillissement cutané du visage.

4. Procédé selon l'une quelconque des revendications 1 à 3, destiné à augmenter la synthèse d'élastine et/ou de collagène dans le derme.

5. Procédé selon l'une quelconque des revendications 1 à 4, dans lequel le produit dérivé de fibroblastes gingivaux est administré par voie topique ou par injection intradermique.

6. Procédé selon l'une quelconque des revendications 1 à 5, dans lequel le produit dérivé de fibroblastes gingivaux est un milieu conditionné de fibroblastes gingivaux.

7. Procédé selon l'une quelconque des revendications 1 à 6, dans lequel le produit dérivé de fibroblastes gingivaux est obtenu à partir de fibroblastes gingivaux prélevés sur l'individu.

8. Procédé selon l'une quelconque des revendications 1 à 7, comprenant :
- le prélèvement de fibroblastes gingivaux sur l'individu ;
- la culture des fibroblastes gingivaux ;
- l'obtention d'un produit dérivé de fibroblastes gingivaux à partir de la culture de fibroblastes gingivaux, ledit produit dérivé de fibroblastes gingivaux étant choisi parmi le groupe comprenant des cellules entières de fibroblastes gingivaux, une culture de fibroblastes gingivaux et un milieu conditionné de fibroblastes gingivaux; et l'administration du produit dérivé de fibroblastes gingivaux à l'individu.
